(19) **Europäisches Patentamt European Patent Office Office européen des brevets**

(11) **EP 1 689 908 B1**

(12) **FASCICULE DE BREVET EUROPEEN**

(45) Date de publication et mention de la délivrance du brevet:
**05.09.2012 Bulletin 2012/36**

(51) Int Cl.:
*C23C 16/50* (2006.01)   *B05D 7/24* (2006.01)

(21) Numéro de dépôt: **04798919.9**

(22) Date de dépôt: **19.11.2004**

(86) Numéro de dépôt international:
**PCT/IB2004/003795**

(87) Numéro de publication internationale:
**WO 2005/049886 (02.06.2005 Gazette 2005/22)**

(54) **DISPOSITIF DE DEPOT DE FILM MINCE PAR PLASMA**

PLASMA-DÜNNFILMABSCHEIDUNGSVORRICHTUNG

PLASMA THIN-FILM DEPOSITION APPARATUS

(84) Etats contractants désignés:
**AT BE BG CH CY CZ DE DK EE ES FI FR GB GR HU IE IS IT LI LU MC NL PL PT RO SE SI SK TR**

(30) Priorité: **20.11.2003 EP 03026470**
**02.02.2004 EP 04002256**

(43) Date de publication de la demande:
**16.08.2006 Bulletin 2006/33**

(73) Titulaire: **Apit Corp. SA**
**1950 Sion (CH)**

(72) Inventeurs:
• **KOULIK, Pavel**
**F-67113 Blaesheim (FR)**

• **KRAPIVINA, Svetlana**
**F-196620 Saint Petersbourg (FR)**
• **MUSIN, Nail**
**F-67400 Illkirch-Graffenstaden (FR)**
• **SAITCHENKO, Anatoli**
**F-67400 Illkirch-Graffenstaden (FR)**
• **ZORINA, Evguenia**
**F-67113 Blaesheim (FR)**

(74) Mandataire: **Vigand, Philippe et al**
**Novagraaf International SA**
**Chemin de l'Echo 3**
**1213 Onex (CH)**

(56) Documents cités:
**WO-A-00/47798      WO-A-02/23960**
**US-A- 5 677 010      US-A1- 2003 104 141**

**EP 1 689 908 B1**

**Description**

La présente invention concerne un dispositif de dépôt d'un film mince sur une surface à traiter par plasma. L'invention concerne notamment le dépôt de films organiques ou polymériques, plus particulièrement pour l'imperméabilisation de la surface à traiter.

Des procédés pour le dépôt d'un film mince par plasma sur la surface d'un objet à traiter sont déjà connus ou utilisés dans divers domaines, par exemple pour le dépôt de couches minces sur des semi-conducteurs, tel que décrit dans le brevet américain US 5,569,502, pour le dépôt de couches barrières ou d'imperméabilisation sur la surface de bouteilles en PET, tel que décrit dans la demande internationale PCT/IB02/01001, pour le dépôt de films sur des textiles, tel que décrit dans le brevet européen EP 1 024 222, pour le dépôt de films imperméables comestibles pour des aliments, tel que décrit dans la demande WO 96/22030.

Dans beaucoup de ces applications, on cherche à déposer des couches très minces en vue des avantages que celles-ci procurent, tels que la flexibilité, la transparence, la possibilité de former des multicouches, l'absence d'influence sur les caractéristiques générales de l'objet à traiter, ou encore pour d'autres propriétés.

Dans le cas de produits ingérables, tels que les produits alimentaires ou pharmaceutiques, une couche très mince a l'avantage de passer inaperçue par le consommateur.

Dans le domaine textile, les couches très minces ont l'avantage d'être très flexibles et de pouvoir maintenir leur intégrité malgré les déformations importantes que doivent subir les objets faits de tissu, tels que les vêtements. Il est cependant important que cette couche soit très uniforme afin de garantir ses propriétés d'imperméabilisation ou d'autres propriétés voulues sur toute la surface à traiter.

Les procédés de dépôt d'un film mince par plasma concernent souvent le dépôt de films inorganiques, résultant de réactions plasmochimiques de particules (ions, atomes, radicaux, particules activées) provenant d'un gaz ou d'un mélange de gaz précurseurs activés par un plasma. La plupart des procédés proposés s'effectuent sous vide partiel pour pouvoir mieux maîtriser les paramètres du plasma et les conditions de développement de la couche mince. Les procédés à vide sont toutefois extrêmement onéreux pour des applications industrielles et la production en série. En outre, la présence d'un vide a le désavantage d'influer sur les propriétés de l'objet à traiter, (par exemple, en déshydratant les objets à traiter). Un autre problème rencontré dans la formation d'un film mince par des procédés de plasma connus, est la création de poudre non désirée sur la surface traitée.

Bien que les films inorganiques soient souhaités dans de nombreuses applications, il y aura également un avantage de pouvoir former des films minces en matières organiques ou polymériques, surtout, mais pas seulement, dans le domaine des produits ingérables, tels que les produits comestibles ou pharmaceutiques. En effet, même si certains matériaux inorganiques sont autorisés dans des aliments en petites quantités, et proposés par exemple dans la demande WO 9622030, il y a une volonté générale d'éviter leur utilisation tant que possible. La présence dans l'organisme humain d'éléments tels que des débris de films inorganiques, contenant différents métaux sous forme d'oxyde, peut avoir des conséquences néfastes. De surcroît, le procédé décrit dans la demande précitée s'opère par plasma sous vide, procédé qui est onéreux et peu approprié à la production en série. L'expérience montre également que le procédé de dépôt de films inorganiques sur des surfaces à traiter d'aliments, de tissus, du papier et d'autres objets, dont la géométrie et les propriétés de surface peuvent varier relativement fortement d'un objet à l'autre, est extrêmement difficile à maîtriser de manière fiable.

En ce qui concerne le domaine alimentaire, les couches de protection contre l'oxydation (barrière à l'oxygène) ou contre l'absorption ou la perte d'humidité (barrière à l'eau et la vapeur d'eau) sont très recherchées et divers procédés de dépôt de films organiques, tels que les couches de cellulose, polydextrose, de lipides ou de protéines, sont proposés dans l'état de la technique. Les procédés connus de dépôt de films organiques sur des produits comestibles s'opèrent toutefois sans plasma et résultent dans des couches dont l'épaisseur n'est pas suffisamment faible pour rester inaperçue ou où les propriétés de barrière de la couche ne sont pas très bonnes ou fiables.

Par exemple, dans la demande de brevet WO 87/03453, on propose d'utiliser des dépôts organiques, comme la cellulose et les couches lipides pour protéger des produits comestibles. Ces couches se déposent sous forme de solution ou de suspension liquide et sont ensuite séchées. Outre la difficulté de déposer un film très mince formant une bonne barrière par un tel procédé, le procédé de séchage est coûteux et peut avoir des effets néfastes sur l'objet à traiter.

Dans le brevet US 6,312,740, on propose de déposer une couche sous forme de poudre comestible chargée électrostatiquement sur la surface des produits alimentaires, une décharge corona étant utilisée pour charger les particules de poudre. Il est mentionné que la dimension idéale des particules est de l'ordre 120 $\mu$m, de telles dimensions ne restant toutefois pas inaperçues par un consommateur.

Il convient de souligner que l'efficacité des couches déposées est déterminée par leur épaisseur, leur densité et leur adhésion à la surface à traiter. Dans le cadre de dépôt d'un film sur des produits comestibles, l'augmentation de l'épaisseur de la couche entraîne une altération du goût du produit protégé. L'expérience montre que l'on ressent des particules dont les dimensions dépassent 1 $\mu$m. Cela revient à exiger qu'une couche protectrice soit ultrafine, si possible moins de 0,5 $\mu$m. Dans le domaine alimentaire on recherche également des couches qui ne contiennent aucun produit toxique, qui n'influencent pas le goût du produit, et pour la plupart des applications qui ne sont pas solubles dans l'eau.

Aucun procédé proposé à ce jour ne répond de manière satisfaisante à ces exigences.

Ces propriétés de couches minces sont également recherchées pour des applications dans d'autres domaines, par exemple pour l'imperméabilisation de textiles ou de papier, le dépôt d'un film sur des bouteilles et emballages, notamment dans les domaines alimentaire ou pharmaceutique, ou encore dans d'autres applications.

Au vu de ce qui précède, un but de l'invention est de fournir un dispositif de dépôt d'un film sur une surface à traiter, le film étant très mince, uniforme et efficace, le dispositif étant adapté à une production industrielle, notamment le traitement d'objets produits en grande série.

Il est avantageux que le dispositif pour la mise en oeuvre du procédé ne soient pas onéreux.

Il est avantageux de fournir un dispositif pour la mise en oeuvre du procédé qui permettent de déposer, de manière fiable, des couches minces sur des objets de différentes formes et dimensions ou ayant des surfaces complexes.

Il est avantageux de fournir un dispositif pour le dépôt d'un film mince qui ne contient pas ou peu de composants inorganiques, notamment dans les domaines alimentaire et pharmaceutique.

Il est avantageux de fournir un dispositif pour le dépôt d'un film sur divers aliments ou produits pharmaceutiques ou autres produits ingérés pouvant être mis en oeuvre dans une chaîne de production à un coût raisonnable.

Il est avantageux de fournir pour certaines applications un dispositif pour le dépôt d'une couche très mince, en particulier inférieure à 1 $\mu$m, en matière organique ou polymérique sur la surface à traiter de divers matériaux.

Un autre but de l'invention est de fournir un dispositif pour le dépôt d'un film imperméabilisant pour des aliments, produits pharmaceutiques ou autres produits ingérables, qui est efficace, fiable et non nocif.

Un autre but de l'invention est de fournir un dispositif pour le dépôt de revêtements organiques ou polymériques très minces, uniformes et efficaces, notamment des revêtements formant barrière à des liquides ou des gaz.

Des buts de l'invention sont réalisés par le dispositif selon la revendication 1.

Dans la présente invention, le processus de dépôt de film est basé sur la génération d'un plasma dans un mélange de gaz plasmagène inerte et de gaz précurseurs et sur la projection du gaz (plasma) formé sur la surface à traiter. Les gaz précurseurs comprennent au moins deux composantes.

Une première composante comprend des substances organiques saturées ou d'un mélange de substances organiques saturées.

Une deuxième composante comprend des substances organiques non-saturées ou un mélange de substances organiques non-saturées.

Dans la composition des gaz précurseurs rentrent le carbone et/ou l'hydrogène et/ou des éléments halogéniques.

Les substances organiques non-saturées sont la source principale de radicaux multivalents libres lourds. Ceux-ci constituent principalement les blocs de construction de la chaîne polymérique.

Les substances organiques saturés, suite aux procédés plasmochimiques dans la zone de plasma, se trouvent être la source principale de radicaux libres légers, avec une seule liaison libre. Ces radicaux sont responsables de l'arrêt du développement de la chaîne polymérique.

Les radicaux multivalents lourds adsorbent davantage à la surface. Cela fait que les rapports entre les quantités de blocs de construction et des radicaux à une liaison seront différents sur la surface à traiter et dans le volume. D'une part cela va donner une chaîne polymérique écourtée et donc une limitation à la formation de poudres en volume, et de l'autre, un dépôt intensifié de film à la surface.

Le dépôt de film est stimulé par une activation préalable de la surface.

Un autre avantage important de ce procédé utilisant le dispositif selon l'invention est qu'il peut être effectué à pression atmosphérique et ne nécessite donc aucune pompe, ni enceinte à vide, simplifiant par conséquent les équipements pour la mise en oeuvre du procédé en milieu industriel, notamment pour le traitement d'objets en série. L'utilisation de deux gaz précurseurs ou mélanges de gaz précurseurs permet également d'obtenir une vitesse de dépôt de film élevée, plus élevée que les dépôts réalisés par des procédés de plasma à vide.

Le dispositif selon l'invention a également l'avantage de placer la décharge électrique pour la création du plasma à une distance relativement éloignée de la surface à traiter, ce qui permet d'optimiser le rendement du procédé et d'éviter le problème de surchauffe de l'objet à traiter. En effet, les radicaux issus des au moins deux gaz précurseurs ou mélanges de gaz précurseurs passent dans le plasma et vivent un certain temps, correspondant au temps maximal de migration pour amener les radicaux à la surface à traiter pour la réaction plasmochimique avec celle-ci. Cette distance optimale est également déterminée par la nécessité d'activer la surface à traiter par les particules ionisées et le rayonnement ultraviolettes et par les atomes activés formés par le plasma.

Pour l'obtention de films polymériques protecteurs, sur la surface de produits, par exemple, alimentaires, on peut avantageusement utiliser comme précurseurs des gaz formés de $C_kH_l$, $C_mF_n$ ou $C_sH_pF_r$, ou de leurs mélanges. La formation du film protecteur est conditionnée par des procédés plasmochimiques en volume et en surface.

Les procédés en surface ont lieu sur la surface de l'objet à traiter, en plusieurs stades :

• Formation de centres de développement sur la surface à traiter, préalablement activée par le plasma. Cette surface peut être lisse ou rugueuse on même fortement accidentée, puisque le milieu activé agissant sur la surface à pression atmosphérique, surtout hors d'équilibre thermodynamique, peut avoir une très faible viscosité et qui conditionne la pénétration des composants actives entre les aspérités de la surface.

• Naissance (initiation) de chaînes dues au dépôt de radicaux libres multivalents actifs, provenant du gaz ou mélange de gaz précurseur lourd, sur la surface à traiter :

$$\text{centre de développement} \; + \; M^{\cdot} \; \rightarrow \; M_1^{\cdot}$$

• Développement de chaînes :

$$M_1^{\cdot} \; + \; M^{\cdot} \; \rightarrow \; M_2^{\cdot}$$

$$M_2^{\cdot} \; + \; M^{\cdot} \; \rightarrow \; M_3^{\cdot}$$

$$M_3^{\cdot} \; + \; M^{\cdot} \; \rightarrow \; M_4^{\cdot} \quad \text{etc.}$$

• Rupture de la chaîne par les radicaux monvalents provenant du gaz ou mélange de gaz précurseur léger :

$$M_n^{\cdot} \rightarrow P_n, \text{ où } P_n, \text{ est un polymère non actif.}$$

De cette manière, on voit que la formation de la couche polymérique sur la surface du produit à traiter comprend la formation de « blocs de construction » dans la zone de plasma, la migration de ces blocs vers la surface, et la formation d'une couche polymérique ne contenant pas de radicaux libres, sur la surface.

Il y a, bien sûr, une concurrence entre la formation de poudres polymériques, en volume, et la formation de la couche polymérique en surface. Cette concurrence est résolue notamment par le choix des composantes du mélange des gaz organiques précurseurs.

Par exemple, on utilise avantageusement un mélange d'une composante non-saturée telle que l'hexafluorpro-pylène ($C_3F_6$), et d'une composante saturée telle que le tétrafluorméthane ($CF_4$).

Le $CF_4$, passant dans le plasma, forme, notamment,des radicaux légers $CF_3^{\cdot}$. Ce sont ces radicaux qui arrêtent la formation des chaînes polymériques.

Le $C_3F_6$ forme les biradicaux lourds $(CF_3\text{-}CF\text{-}CF_2)^{\cdot\cdot}$ qui sont les blocs de fabrication des polymères.

En principe, ces réactions ont lieu en volume et sur la surface traitée. La concurrence entre la formation des polymères en volume et en surface est déterminée par le rapport entre les concentrations des radicaux lourds et légers. A la surface, la concentration des biradicaux lourds prévaut sur celle des radicaux légers, a qui avantage la formation du film polymérique sur la surface traitée.

En volume, la concentration des radicaux légers $CF_3^{\cdot}$ prévaut sur celle des radicaux lourds. Il en résulte que la formation des poudres polymérique y est freinée.

En variant les proportions de ces composantes de manière empirique, les expériences réalisées dans le cadre de l'invention montrent qu'on peut pratiquement exclure la formation de poudre en volume.

L'utilisation de précurseurs fluoroorganiques, comme dans l'exemple précité, permet la formation d'un film se polytetrafluoréthylène ($\text{-}CF_2\text{-}CF_2\text{-})_n$ sur la surface à traiter.

Le film de polytetrafluoréthylène (aussi connu couramment sous le nom "teflon") est un film ayant de très bonnes propriétés imperméabilisantes par rapport à l'eau et à l'oxygène et est un matériau non nocif, et donc bien adapté à des utilisations dans le domaine des produits comestible, que ce soit pour le dépôt d'un film sur des produits alimentaires ou sur les emballages pour le conditionnement de produits alimentaires ou de boissons.

D'autres précurseurs halogéno-organiques peuvent aussi être utilisés pour la formation de couches polymériques sur une surface à traiter. La présence du chlore dans les produits ingérables n'étant toutefois, d'une manière générale, pas désirée, les couches polymériques contenant du chlore sont à utiliser dans les domaines non alimentaires.

On peut également utiliser, en tant que gaz précurseur, des hydrocarbures permettant de former des films polymériques sur la surface à traiter, notamment du polyéthylène ramifié ($\text{-}CH_2\text{-}CH_2\text{-})_n$.

Si le produit précurseur est un mélange contenant du carbone, du fluor (ou autre halogène) et de l'hydrogène, on a une réaction de co-polymérisation.

Un avantage important du dispositif selon l'invention est qu'il est possible de bien maîtriser la formation de couches polymériques sur la surface de l'objet à traiter en évitant la croissance en volume et, par conséquent, d'obtenir des couches d'épaisseur très uniformes. La grande uniformité des couches déposées implique que des couches très minces, et même monomoléculaires, c'est-à-dire de l'ordre de 0.5 nanomètres, sont bien réparties sur la surface à traiter. Comme le développement de la couche sur la surface à traiter se passe en surface en non en volume, des couches avec des épaisseurs plus grandes peuvent également être déposées avec une grande uniformité, l'épaisseur des couches pouvant être contrôlée notamment en variant le temps de traitement. Il est également possible dans le cadre de l'invention de former un film multicouche, c'est-à-dire formé de couches de différentes compositions en injectant différents gaz précurseurs séquentiellement, lors du traitement par plasma. Le mécanisme permettant le développement des couches polymériques en surface a également l'avantage d'éliminer les radicaux libres, ce qui permet l'utilisation du procédé pour des produits comestibles. En résumé, les films polymériques formés à l'aide du dispositif selon l'invention sont chimiquement stables, biocompatibles, imperméables aux gaz et à l'eau, ont une haute élasticité et solidité mécanique, et ont de bonnes qualités diélectriques. Leur épaisseur peut varier suivant les contraintes de l'application entre 0,5 nm (couche monomoléculaire) et 0,5 $\mu$m, épaisseur au-dessus de laquelle le film déposé peut se désagréger et se séparer de la surface traitée.

Le plasma généré, selon l'application, fonctionne en régime continu ou par impulsions. Il est alimenté par une source de courant continu, ou alternatif ou à haute fréquence ou en micro-ondes. Le plasma généré est spécialement efficace en régime par impulsions.

Le procédé peut avantageusement être mis en oeuvre en utilisant un plasma à pression atmosphérique, ce qui permet d'intégrer relativement facilement le dispositif de traitement dans une chaîne de production industrielle et de réaliser le dépôt de film à des cadences industrielles

A l'aide du dispositif selon l'invention, on génère de préférence un plasma hors d'équilibre thermodynamique, ce plasma étant propice à la formation efficace de couches, mêmes très fines, sur la surface à traiter à pression atmosphérique. On peut avantageusement contrôler les impulsions de la décharge, afin de générer des ondes de choc qui génèrent des vibrations ultrasoniques améliorant les réactions plasmochimiques et détruisant la couche limite hydrodynamique sur la surface à traiter. Tous ces éléments concourent à accélérer le procédé de dépôt de film et d'en augmenter son efficacité.

Les gaz précurseurs, porteurs des éléments composant le film, sont introduits dans la zone de dépôt du film soit simultanément notamment sous forme de mélange, soit consécutivement sous forme de portions de différents mélanges gazeux.

Dans le premier cas, la composition optimale des quantités des différents gaz précurseurs peut être atteinte empiriquement.

Dans le deuxième cas, il est possible, tout en conservant l'opportunité de déposer des films de composition optimale contenant un minimum de poudres ou n'en contenant pas, de former des films multicouche qui assurent non seulement l'imperméabilité du film mais sa flexibilité, sa solidité et d'autres propriétés (mécaniques, optiques, chimiques). Dans ce cas, le film résultant a une composition variable sur son épaisseur. En particulier, le film peut être considéré comme multicouche.

Les paramètres du processus de dépôt de film, et notamment les paramètres de génération du plasma et l'activation de la surface à traiter, peuvent être adaptés de manière à tuer les micro-organismes qui se trouvent sur la surface traitée au moment du traitement. Cette propriété de la présente invention est particulièrement utile lors du traitement des produits alimentaires. Lors de ce traitement, ces produits sont non seulement recouverts d'un film protecteur empêchant la migration des gaz (en particulier de l'oxygène) des vapeurs (en particulier des vapeurs d'eau) et de l'eau en phase liquide, mais aussi de celle des microorganismes tels que, par exemple, les moisissures ou les levures apportées de l'atmosphère ambiante ou des contacts extérieurs.

Des dispositifs pour la mise en oeuvre du procédé et des exemples de traitement seront maintenant décrits à l'aide des dessins dans lesquels:

la Fig. 1 est un schéma simplifié, illustrant un dispositif de plasma à deux buses pour le traitement local avec balayage de la surface à traiter dans deux dimensions;

la Fig. 2 est un schéma simplifié, illustrant un dispositif de plasma selon l'invention, destiné à être inséré dans une chaîne de production industrielle;

la Fig. 3 est un schéma simplifié, illustrant une autre variante d'un dispositif de plasma à insérer dans une chaîne de production industrielle;

la Fig. 4 est un schéma simplifié, illustrant une autre variante d'un dispositif de plasma à insérer dans une chaîne de production industrielle;

la Fig. 5 est un schéma simplifié, illustrant une autre variante d'un dispositif de plasma à insérer dans une chaîne de production industrielle;

les Figures 6 à 11 sont des photographies de produits alimentaires non traités et traités, illustrant l'imperméabilité à l'eau des produits traités par des procédés de plasma selon l'invention par rapport aux produits non traités;

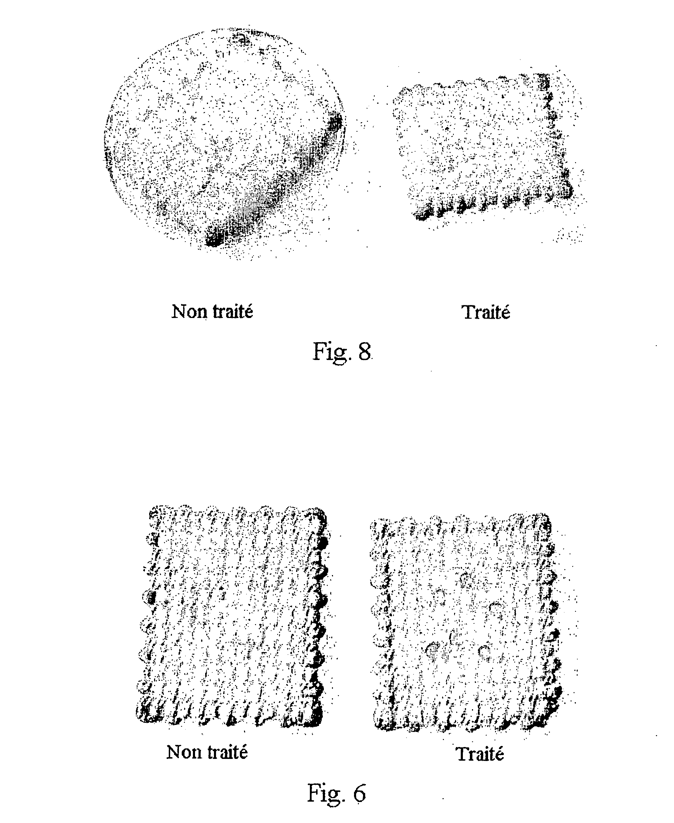

la Fig. 6 représente des gouttes d'eau colorée sur biscuits secs;

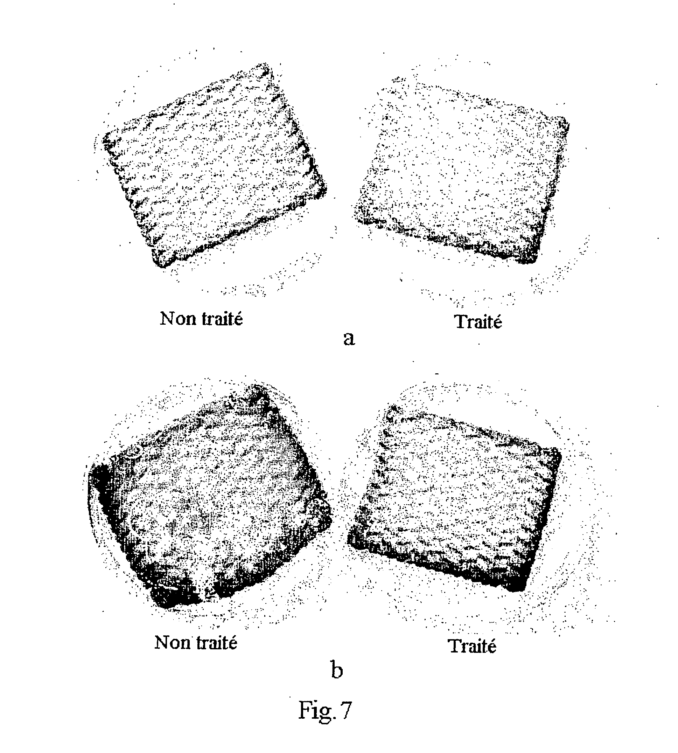

la Fig. 7 représente des biscuits secs dans l'eau, la Fig. 7a étant une photo faite immédiatement après immersion, et la Fig. 7b étant une photo faite 20 sec après immersion;

la Fig. 8 représente des biscuits secs dans l'eau, la photo étant faite 30 min après immersion;

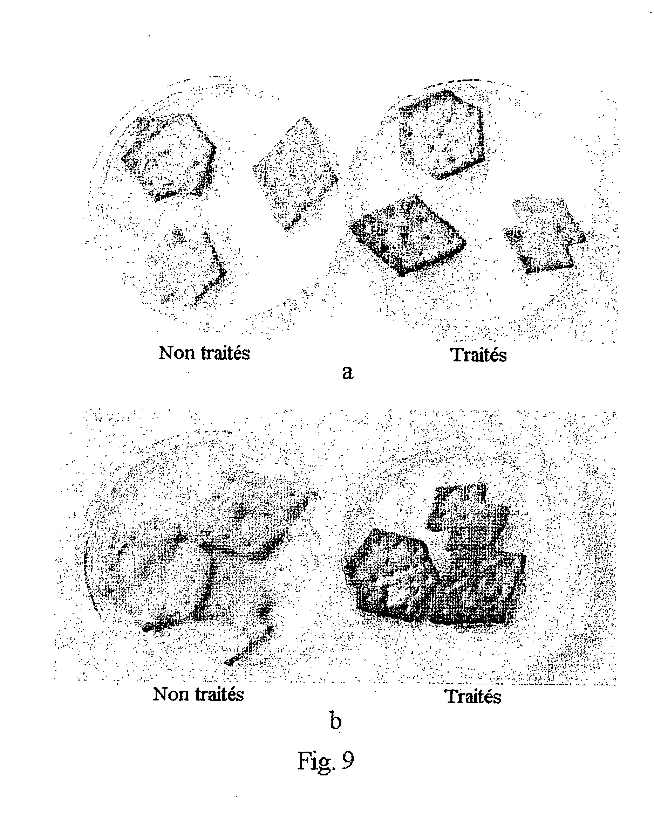

la Fig. 9 représente des crackers dans l'eau, la Fig. 7a étant une photo faite immédiatement après immersion, et la Fig. 7b étant une photo faite 20 sec après immersion;

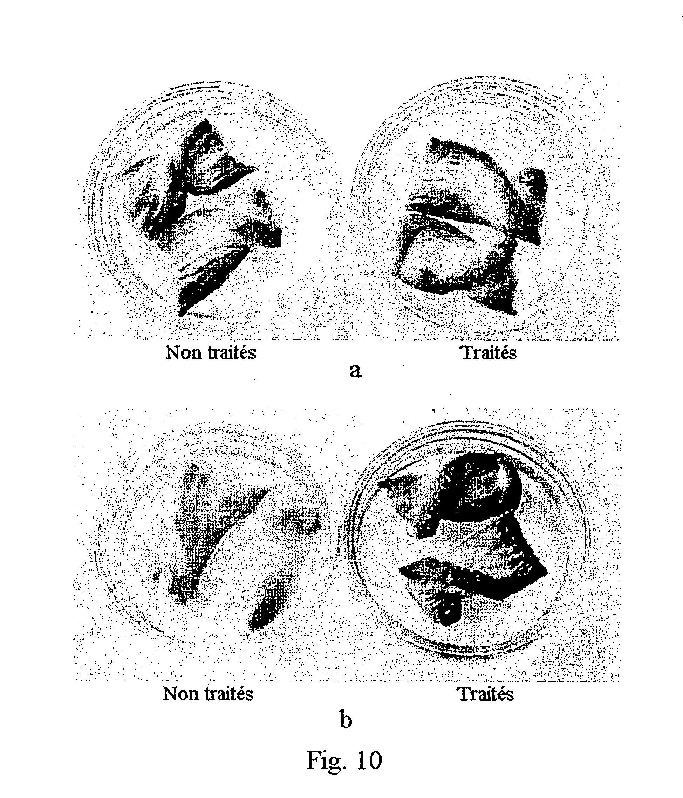

la Fig. 10 représente des snacks feuilletés au beurre dans l'eau, la Fig. 7a étant une photo faite immédiatement après immersion, et la Fig. 7b étant une photo faite 40 min après immersion; et

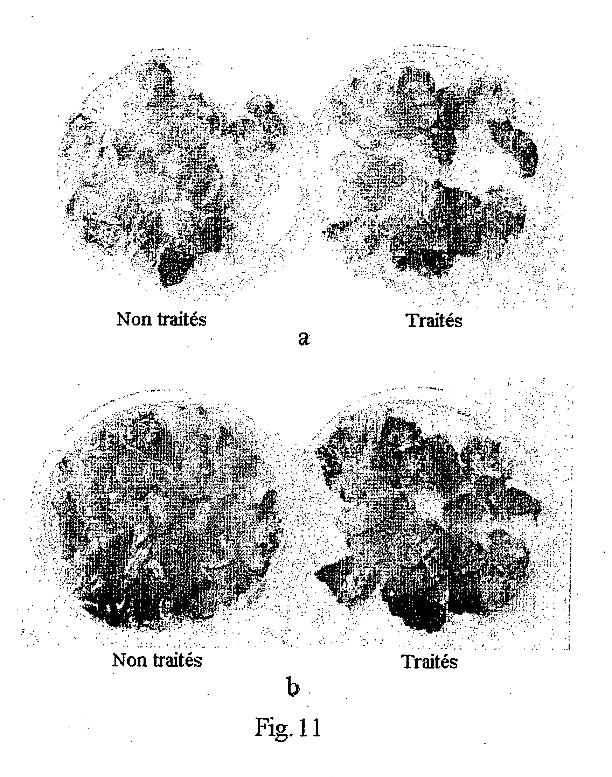

la Fig. 11 représente des corn flakes dans l'eau, la Fig. 7a étant une photo faite immédiatement après immersion, et la Fig. 7b étant une photo faite 1 h après immersion.

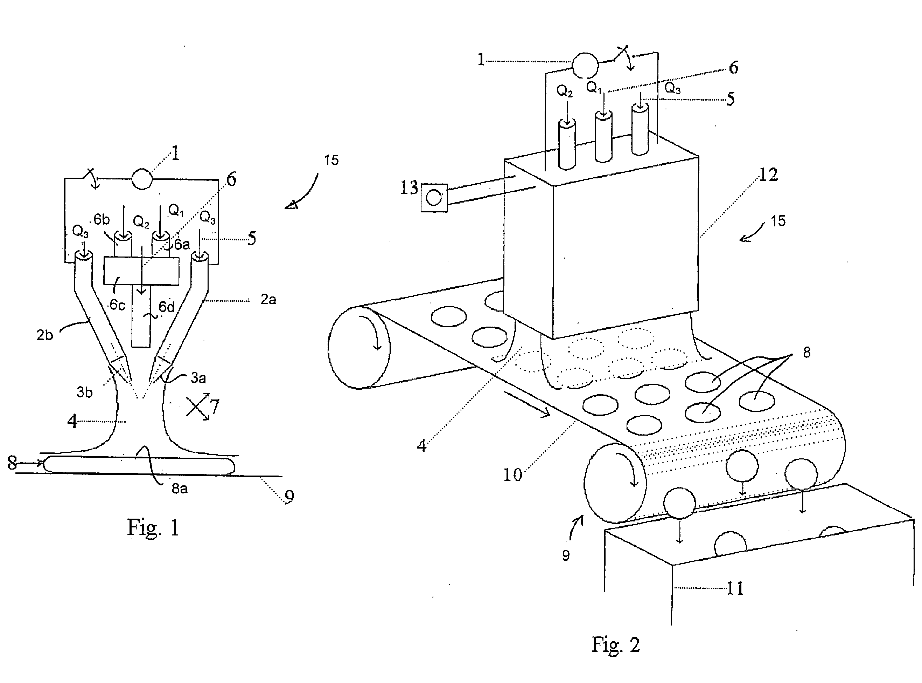

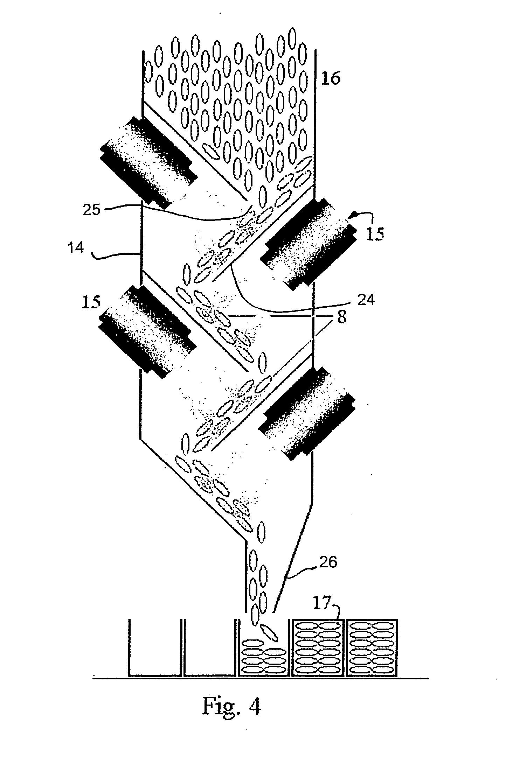

Faisant référence aux Figures 1 à 5, un dispositif de traitement de surface par plasma pour la mise en oeuvre d'un procédé selon l'invention comprend un ou plusieurs générateurs de plasma 15, produisant un plasma 4 appliqué sur la surface à traiter 8, en déplacement par rapport au générateur de plasma, soit au moyen d'un système cinématique 9 tel qu'un convoyeur 10 (Figures 2, 3 et 5), ou débitant par force de gravitation à travers un réacteur 14 (Fig. 4) soumis par exemple à des vibrations afin d'assurer le flux contrôlé des objets à traiter à travers le réacteur.

Faisant référence plus particulièrement aux Figures 1 et 2, le générateur de plasma 15 comprend une source de courant 1, par exemple une source de courant alternatif 50 Hz avec une tension nominale de 1000 V, connectée à des électrodes 2a, 2b, sous forme par exemple de tubes alimentés à une extrémité par un gaz d'apport $Q_3$ et ayant, à l'autre extrémité, des buses 3a, 3b pour diriger et influencer la forme du plasma généré 4. Le générateur de plasma 15 comprend en outre un système d'alimentation 6 des gaz précurseurs $Q_1$, $Q_2$, ce système comprenant des conduits d'entrée 6a, 6b, un mélangeur 6c, et un conduit de sortie 6d. Le système d'alimentation en gaz précurseur peut également être un dispositif distinct et séparé du générateur de plasma, mais il est avantageux d'intégrer ce dispositif dans le générateur ou bloc de générateurs, afin de pouvoir mieux maîtriser le passage des gaz précurseurs dans le plasma et l'apport des composants résultant sur la surface à traiter 8a. Le conduit de sortie des gaz précurseurs 6d ainsi que les buses du gaz d'apport 3a, 3b permettent de diriger et de contrôler la forme géométrique du flux de plasma 4 sur le support 9 transportant les objets à traiter.

Le support 9 et/ou le générateur 12 peuvent être montés sur un système cinématique effectuant un mouvement relatif en une ou plusieurs dimensions, afin que le flux de plasma 4 balaye les objets à traiter. Le système cinématique peut être un convoyeur dans une chaîne de production industrielle, tel que montré dans la Fig. 2 et les Figures 3 et 5, où les objets à traiter 8 passent à travers le plasma 4 projeté vers la surface du convoyeur.

Dans le dispositif selon la Fig. 2, afin de former un rideau linéaire de plasma 4 ayant essentiellement la largeur du convoyeur sur lequel sont placés les objets à traiter, plusieurs générateurs de plasma peuvent être montés de manière juxtaposée dans un bloc de générateurs 12. Le bloc de générateurs a des conduits pour l'alimentation du gaz d'apport et précurseurs, une source de courant et un contrôleur à microprocesseur 13 couplé à la source de courant, afin de contrôler les paramètres d'alimentation en énergie électrique des générateurs de plasma.

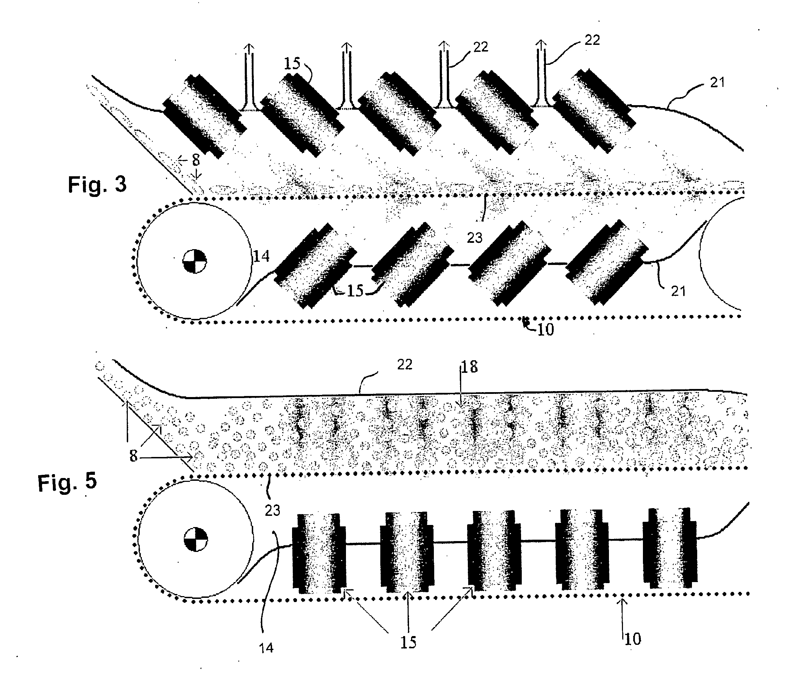

Faisant référence à la Fig. 3, il est également possible de disposer des générateurs de plasma, sous forme de plasmotrons, le long du convoyeur de part et d'autre de la bande 21 du convoyeur, le plasma étant enfermé dans une enceinte d'un réacteur délimitée par des parois 21 et comprenant des conduits de ventilation 22 des gaz résiduels. Dans ce cas, la bande 23 du convoyeur peut être sous forme de grille ou de mèche métallique afin de permettre le traitement par le plasma généré de part et d'autre de la bande 23 sur toute la périphérie de l'objet à traiter. Dans le cas du traitement de surface d'objets relativement petits ou légers, tels que montrés dans la Fig. 5, les générateurs de plasma 15 peuvent être disposés sous les objets à traiter, les forces hydrodynamiques des jets de plasma étant utilisées pour soulever et brasser les objets à traiter, assurant ainsi un traitement de surface par plasma sur toute la surface de l'objet.

Le brassage et le retournement des objets transportés sur un convoyeur peuvent également être effectués en faisant vibrer la bande du convoyeur et, dans ce cas, il est possible de ne prévoir des générateurs de plasma que du côté supérieur de la bande.

Au lieu d'utiliser un système cinématique pour le déplacement des objets à traiter, on peut également les débiter à travers les flux de plasma dans une enceinte de réacteur 14 par gravitation, ou par force hydrodynamique (par exemple, air soufflé). Le réacteur 14 peut être muni d'éléments de guidage, par exemple sous forme de parois 24, pour diriger les objets à traiter 8 successivement dans les flux de plasma projetés par les générateurs de plasma 15. L'enceinte de réacteur 14 est de préférence vibrée afin d'assurer l'écoulement des objets à traiter à travers les orifices 25 formés par des éléments de guidage et servant à contrôler le débit d'objets à traiter à travers l'enceinte 14. L'évacuation des produits à traiter peut être dirigée dans un système de conditionnement 17 monté sur un système cinématique.

Les générateurs de plasma sont de préférence placés dans l'enceinte d'un réacteur composé d'un boîtier qui isole le plasma hydrodynamiquement de l'environnement extérieur, afin de mieux contrôler les flux de plasma sur les objets à traiter.

Le générateur de plasma peut être un générateur à courant continu, à courant alternatif, ou à haute fréquence, ou un générateur à micro-ondes. De préférence, le générateur est conçu de manière à alimenter la décharge de plasma en impulsions électriques. Dans ce cas, il comprend de préférence un dispositif hacheur dans un microprocesseur 13 permettant de former les impulsions en amplitude et en durée suivant les contraintes du procédé.

Des exemples de traitement de surface d'objets et notamment de produits comestibles, utilisant des dispositifs selon l'invention, sont présentés ci-après.

## **Exemple 1**

Le produit alimentaire à traiter est un biscuit sec, connu couramment sous la désignation «Petit Beurre». Le produit a été placé dans le réacteur d'un dispositif correspondant à la Fig. 1. Le traitement est effectué à pression atmosphérique.

Les paramètres du procédé étaient les suivants :

1. Gaz plasmagène : argon

2. Gaz précurseur : tetrafluorméthane (50%) et hexafluoropropylène (50%)

3. L'alimentation de la décharge s'effectue par une source de courant alternatif à haute tension. Le plasmotron est un générateur de plasma constitué de deux électrodes sous forme de buses par lesquels les gaz arrivent dans la zone de plasma.

4. Tension de mise en marche de la décharge : 10 kV

5. Tension pendant la décharge : $1 \div 2{,}5$ kV

6. Courant: 100 mA

7. Les gaz plasmagène et précurseurs sont projetés dans la zone de décharge par les buses des électrodes

8. Le débit total de gaz est $\leq 5$ l/min

9. La distance entre les électrodes et la surface traitée est de ~ 4 cm

10. La vitesse linéaire de la surface traitée par rapport au plasma est ~ 1 m/min

11. La vitesse de balayage est de 10 cm/min

12. La durée du traitement : 30 sec

Résultats:

La composition du film déposé, qui est vérifiée par spectroscopie infrarouge, est du tetrafluorethylène (téflon), l'épaisseur moyenne de la couche étant 30nm.

A l'aide de la méthode de spectroscopie électronique paramagnétique, il a été constaté qu'il n'y a aucune trace de radicaux libres résiduels sur la surface traitée.

Les biscuits traités ont été testés par rapport à la perméabilité à l'eau. Les gouttes d'eau déposées à la surface du biscuit non traité sont immédiatement absorbées. Les gouttes d'eau déposées sur la surface du biscuit traité ne s'absorbent pas. Elles roulent sur la surface sans être absorbées, tel que montré dans la Fig. 6.

Les biscuits traités et non traités ont été immergés dans l'eau. Le biscuit traité s'est immédiatement imbibé d'eau, tel que montré dans la Fig. 7.

Le biscuit traité n'a commencé à absorber lentement de l'eau qu'après 30 min, tel que montré dans la Fig. 8.

L'augmentation du poids du biscuit immergé dans l'eau pendant 5 sec était comme suit:

- Non-traité : 88 % ;
- Traité : 0,7 %.

## Exemple 2

Le produit à traiter était les «Snacks feuilletés au beurre», tels que montrés dans la Fig. 10.

Ils ont été traités à l'aide d'un dispositif tel que montré à la Fig. 1.

Les paramètres du procédé sont ceux de l'exemple 1.

Gaz précurseur : méthane - 60%, acétylène - 40%

Résultats:

L'épaisseur du film obtenu était 100 nm. Le film obtenu est constitué de polyéthylène. Les tests de perméabilité à l'eau ont montré que le produit non traité s'imbibe d'eau en 1 minute. Le produit traité ne s'imbibait pas d'eau après 40 min. après son immersion, tel que montré dans la Fig. 10.

L'augmentation du poids d'un produit non traité et d'un produit traité immergés dans l'eau pendant 30 sec était :

- Non-traité : 91 % ;
- Traité : 0,8 %.

## Exemple 3

Le produit traité était des "Corn Flakes", tel que montré dans la Fig. 11. Le dispositif de traitement par plasma utilisé était du type correspondant à la Fig. 4.

Les paramètres du traitement étaient:

1. Gaz de formation du plasma : argon

2. Gaz précurseurs : tetrafluorméthane - 70% + tetrafluorétylène - 30%

3. Le générateur de plasma est un générateur à courant à haute fréquence (f = 13,56 MHz)

4. Puissance du générateur de plasma : 20 kW

5. Durée du traitement : 30 sec.

Résultats:

Les flocons de «Corn Flakes» traités étaient recouverts d'un film de teflon de 30 à 40 nm d'épaisseur. Après 5 min, les «Corn Flakes» non traités, immergés dans l'eau, perdaient leur forme et leurs propriétés, tel que montré dans la Fig. 11a.

Les «Corn Flakes» traités n'avaient pas perdu leur forme et leurs propriétés 1 heure après leur immersion dans l'eau, tel que montré dans la Fig. 11 b.

## Exemple 4

Le produit traité était des céréales dont le diamètre moyen était de 5 mm. Le traitement était effectué à l'aide d'un dispositif du type montré sur la Fig. 5 avec « couche bouillante ».

Le gaz de formation du plasma (Ar) était envoyé sur le tamis du transporteur mélangé à un gaz précurseur (mélange de méthane - 60 % et d'hexafluoropropylène - 40 %) créant une « couche bouillante » permettant de bien mélanger le gaz réactif au produit.

Le traitement dura 45 sec.

Les produits traités et non traités ont été soumis aux tests d'absorption d'eau. Les produits non traités perdaient leurs propriétés 5 min après leur immersion. Les produits traités ne les perdaient pas une heure après immersion dans l'eau.

### Exemple 5

Le produit traité était un médicament, le Cimetrol 500 LPCI ( Metronidazole) sous forme de comprimés de 500 mg. Ces comprimés sont très sensibles à l'humidité.

Le traitement a été effectué à l'aide d'un dispositif du type montré sur la Fig.5 avec « couche bouillante ».

Le conditions étaient identiques aux conditions de l'exemple 5.

Le traitement dura 20 sec.

Les comprimés traités ont été soumis à des tests d'absorption d'eau. Les comprimés non traités absorbent l'eau en 2 min. Les comprimés traités étaient restés intacts 10 heures après l'immersion.

Le traitement permet d'élever la durée de conservation de ce médicament en blisters de deux mois à plus de deux ans.

### Exemple 6

Le produit traité était un papier de sulfite (glue de résine - 0,5%, alumine - 0,5%, cooling filler - 25%).

L'échantillon (150 x 150 mm) a été placé dans le réacteur d'un dispositif correspondant à la Fig.1.

Les paramètres du procédé sont ceux de l'exemple 1.

> Gaz plasmogène : argon
> Gaz précurseur : mélange méthane (50%) et hexafluorpropylène (50%).

Résultats :

La résistance du papier a été estimée par la stabilité de ses caractéristiques mécaniques lors d'un vieillissement thermique (T = 100 $\pm$ 3°C) pendant 30 jours et une exposition aux rayons ultraviolets des deux faces sous une lampe UV pendant 60 min.

Les tests ont montré que les caractéristiques de solidité et de déformations des échantillons n'ont pratiquement pas changé.

L'imprégnation par capillarité a passé de 36 mm/10 min pour les échantillons non-traités à 0 pour les échantillons traités. L'angle de contact de mouillabilité des échantillons traités est de 125 degrés.

### Exemple 7

Le produit traité était un tissu (densité - 540 g/m$^2$). L'échantillon (150 x 150 mm) a été placé dans le réacteur d'un dispositif correspondant à la Fig.1.

Les paramètres du procédé sont ceux de l'exemple 1.

> Gaz plasmogène : argon
> Gaz précurseur : mélange tetrafluorméthane (75%) et hexafluoropropylène (25%).

Résultats :

Le degré de répulsion de l'huile sur l'échantillon traité a été mesuré égal à 120.

Les gouttes d'eau, déposés sur la surface sèchent sans imprégner le tissu.

La résistance à la colonne d'eau après traitement a augmenté de 0 à 190 cm.

En d'autres termes l'échantillon traité est fortement hydro et oléophobe.

Les propriétés hydrophobes et oléophobe ne sont pas altérées après avoir fait bouillir les échantillons pendant 1 heure dans un eau savonneuse standard.

**Revendications**

1. Dispositif pour la mise en oeuvre d'un procédé de dépôt par plasma d'un film mince sur la surface d'un objet à traiter, **caractérisé en ce qu'**il comporte des générateurs de plasma comprenant des électrodes pour la création de la décharge électrique alimentée par une source de courant, et un système d'alimentation en au moins deux gaz précurseurs, le générateur étant disposé dans une enceinte d'un réacteur (14), le dispositif comprenant en outre un système cinématique pour le transport des objets à traiter à travers le flux de plasma généré par les générateurs, le dispositif travaillant à pression atmosphérique, et **en ce que** le système cinématique du transport des objets à traiter comprennent une bande convoyeur (23) sous forme de mèche ou de grille pour permettre le traitement de surface sur toute la périphérie de l'objet à traiter.

2. Dispositif pour la mise en oeuvre d'un procédé de dépôt par plasma d'un film mince sur la surface d'un objet à traiter, **caractérisé en ce qu'**il comporte des générateurs de plasma comprenant des électrodes pour la création de la décharge électrique, alimentée par une source de courant, et un système d'alimentation en au moins deux gaz précurseurs, le générateur étant disposé dans une enceinte d'un réacteur (14), le dispositif comprenant en outre un système cinématique pour le transport des objets à traiter à travers le flux de plasma généré par les générateurs, le dispositif travaillant à pression atmosphérique, et **en ce que** le dispositif comprend des éléments de guidage (24) guidant le flux d'objets à traiter à travers les flux de plasma des générateurs de plasma disposés le long du réacteur, l'entraînement des objets à traiter se faisant par gravitation ou par flux hydrodynamique.

3. Dispositif selon la revendication 2, **caractérisé en ce que** le traitement s'effectue sur la surface d'objets de petites dimensions, accumulés dans un container à travers lequel, de bas au haut passant les gaz issus du plasma de manier à former une couche bouillante assurant le traitement de la surface entière de chacun des objets.

**Claims**

1. A device for implementing a method for plasma deposition of a thin film on the surface of an object to be treated, **characterized in that** it includes plasma generators including electrodes for creating the electrical discharge supplied by a power source, and a system for supplying it with at least two precursor gases, the generator being placed inside a chamber of the reactor (14), the device also including a kinematic system for transporting objects to be treated through the plasma stream generated by the generators, the device working at atmospheric pressure, and **in that** the kinematic system for transporting objects to be treated includes a conveyor belt (23) in the form of a mesh or a grating to allow surface treatment over the entire periphery of the object to be treated.

2. A device for implementing a method for plasma deposition of a thin film on the surface of an object to be treated, **characterized in that** it comprises plasma generators including electrodes for the creation of an electrical discharge, supplied by a power source, and a system for supplying it with at least two precursor gases, the generator being placed within a chamber of a reactor (14), the device also including a kinematic system for transporting objects to be treated through the plasma stream generated by the generators, the device working at atmospheric pressure, and **in that** the device includes guiding elements (24) guiding the stream of objects to be treated through the plasma streams of the plasma generators arranged along the reactor, the transport of the objects to be treated occurring by gravitation or by hydrodynamic stream.

3. The device according to Claim 2, **characterized in that** the treatment is carried out on the surface of objects of small size, collected in a container through which pass, in an upward direction, the gases emanating from the plasma so as to form a fluidized layer ensuring the treatment of the entire surface of each of the objects.

**Patentansprüche**

1. Vorrichtung zur Durchführung eines Plasmadepotverfahrens eines dünnen Films auf der Oberfläche eines zu bearbeitenden Objekts, **dadurch gekennzeichnet, dass** sie Plasmageneratoren aufweist, die Elektroden für die Bereitstellung der elektrischen Entladung umfasst, die von einer Stromquelle versorgt wird, und ein System zur Versorgung mit mindestens zwei Vorläufergasen, wobei der Generator in einem Reaktorraum (14) angeordnet ist, wobei die Vorrichtung ferner ein kinematisches System zum Transport der durch den von den Generatoren erzeugten Plasmastrom zu bearbeitenden Objekte umfasst, wobei die Vorrichtung bei atmosphärischem Druck arbeitet, und dass das kinematische System für den Transport der zu bearbeitenden Objekte ein Förderband (23) in Form eines Streifens oder eines Rosts umfasst, um die Bearbeitung der Oberfläche über den gesamten Umfang des zu bearbeitenden Objekts zu erlauben.

2. Vorrichtung zur Durchführung eines Plasmadepotverfahrens eines dünnen Films auf der Oberfläche eines zu bearbeitenden Objekts, **dadurch gekennzeichnet, dass** sie Plasmageneratoren aufweist, die Elektroden für die Bereitstellung der elektrischen Entladung umfasst, die von einer Stromquelle versorgt wird, und ein System zur Versorgung mit mindestens zwei Vorläufergasen, wobei der Generator in einem Reaktorraum (14) angeordnet ist, wobei die Vorrichtung ferner ein kinematisches System für den Transport der durch den von den Generatoren erzeugten Plasmastrom zu bearbeitenden Objekte umfasst, wobei die Vorrichtung bei atmosphärischem Druck arbeitet, und dass die Vorrichtung Führungsmittel (24) umfasst, die den Strom der Objekte, die durch die Plasmaströme der entlang des Reaktors angeordneten Plasmageneratoren zu bearbeiten sind, lenken, wobei der Antrieb der zu bearbeitenden Objekte durch Schwerkraft oder durch hydrodynamischen Strom erfolgt.

3. Vorrichtung nach Anspruch 2, **dadurch gekennzeichnet, dass** die Bearbeitung auf der Oberfläche von Objekten kleiner Abmessungen erfolgt, die in einem Behälter angesammelt sind, durch den von unten nach oben die vom Plasma ausgehenden Gase derart strömen, dass sie eine gasende Schicht bilden, die die Bearbeitung der gesamten Oberfläche jedes Objekts sicherstellt.

Fig. 2

Fig. 1

Fig. 3

Fig. 5

13

Fig. 4

Non traité        Traité

Fig. 8

Non traité        Traité

Fig. 6

Non traité     Traité

a

Non traité     Traité

b

Fig. 7

Non traités        Traités

a

Non traités        Traités

b

Fig. 9

Non traités      Traités

a

Non traités      Traités

b

Fig. 10

Non traités        Traités

a

Non traités        Traités

b

Fig. 11

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5569502 A
- EP 1024222 A
- WO 9622030 A
- WO 8703453 A
- US 6312740 B